# FASCICULE DE BREVET EUROPEEN

(11) **EP 1 098 895 B1**
(45) Date de publication et mention de la délivrance du brevet: **05.06.2002**
(21) Numéro de dépôt: 99929486.1
(22) Date de dépôt: 15.07.1999
(51) Int. Cl.: C07D 417/12, A61K 31/425

(54) **PROCEDE POUR LA PREPARATION DU SEL POTASSIQUE DU LINTITRIPT**
VERFAHREN ZUR HERSTELLUNG EINES POTASSIUM SALZES VON " LINTITRIPT "
METHOD FOR PREPARING LINTITRIPT POTASSIUM SALT

(30) Priorité: 20.07.1998 FR 9809218
(43) Date de publication de la demande: 16.05.2001
(73) Titulaire: SANOFI-SYNTHELABO, 75013 Paris (FR)
(72) Inventeur: RADISSON, Jo[l, F-31600 Saubens (FR)
(74) Mandataire: Varady, Peter
(86) Numéro de dépôt international: FR9901724
(87) Numéro de publication internationale: WO0005233

(56) Documents cités:
- EP-A- 0 432 040
- WO-A-97/17064

## Description

La présente invention concerne un procédé pour la préparation du sel potassique du 2-[[4-(2-chlorophényl)-2-thiazolyl]carbamoyl]indole-1-acétate ainsi que les sels et les solvates préparés par ce procédé.

L'acide 2-[[4-(2-chlorophényl)-2-thiazolyl]carbamoyl]indole-1-acétique, représenté par la formule (A) 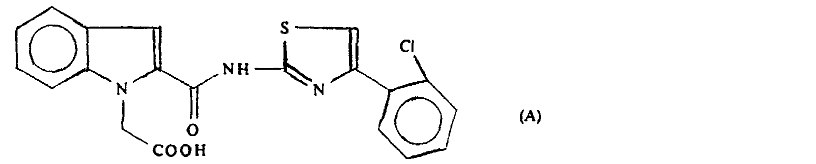 est un puissant antagoniste des récepteurs A de la cholécistokinine (CCK_{A}-antagoniste) décrit pour la première fois dans EP-A-0 432 040 et également connu sous sa Dénomination Commune Internationale "lintitript".

Le lintitript a été étudié sous forme d'acide libre mais, pour la préparation de compositions pharmaceutiques surtout à usage parentérale, l'utilisation de son sel potassique a été considérée comme avantageuse (WO 97/17064).

Dans EP-A-0 432040, le lintitript est préparé par réaction du 2-carboxyindol-1-ylacétate de méthyle avec le 2-amino-4-(2-chlorophényl)thiazole et par saponification du 2-[[4-(2-chlorophényl)-2-thiazolyl]carbamoyl]indole-1-acétate de méthyle avec de l'hydroxyde de sodium et neutralisation avec de l'acide chlorhydrique. Le lintitript ainsi obtenu a cependant l'inconvénient d'absorber de l'acide chlorhydrique, ladite absorption ayant lieu en quantités non stéchiométriques.

Des essais de salification de l'acide 2-[[4-(2-chlorophényl)-2-thiazolyl]carbamoyl]indole-1-acétique avec un hydroxyde alcalin, effectués dans différents solvants, notamment dans de l'acétone ou de l'éthanol, ont conduit à des sels alcalins soit anhydres, soit solvatés, avec un contenu très variable en solvant, eau ou solvant organique. Cette non-reproductivité de la composition des sels n'est pas acceptable pour des produits pharmaceutiques.

De plus, l'utilisation d'hydroxydes alcalins, bases assez fortes, a comme conséquence l'arrachement de plus d'un proton à la molécule (A) ci-dessus et conduit à la formation de mélanges de sels complexes et, encore une fois, peu reproductibles.

Il a été maintenant trouvé que si l'on traite l'acide 2-[[4-(2-chlorophényl)-2-thiazolyl]carbamoyl]indole-1-acétique ou ses esters alkyliques en C₁-C₄ avec du carbonate de potassium dans un solvant constitué par un mélange d'eau et d'un solvant peu miscible à l'eau, le sel potassique du lintitript peut être isolé à l'état pur et sous une forme cristalline bien déterminée par simple filtration.

Il a été enfin trouvé que le sel de potassium du lintitript peut être préparé avec des rendements excellents par saponification *in situ*, avec du carbonate de potassium, d'un 2-[[4-(2-chlorophényl)-2-thiazolyl]carbamoyl]indole-1-acétate de (C₁-C₄)alkyle dans le mélange provenant de la réaction du 1-carboxyindolyl-1-acétate de (C₁-C₄)alkyle avec le 2-amino-4-(2-chlorophényl)thiazole, sans donc isoler l'ester intermédiaire.

Ainsi, selon un de ses aspects, la présente invention concerne un procédé pour la préparation du sel potassique du lintitript, caractérisé en ce que l'on traite l'acide 2-[[4-(2-chlorophényl)-2-thiazolyl]carbamoyl]indole-l-acétique ou ses esters alkyliques en C₁-C₄ avec du carbonate de potassium dans un système solvant biphasique constitué par un mélange d'eau et d'un solvant organique peu miscible à l'eau, éventuellement on recristallise dans l'eau le solvate intermédiaire précipité et on isole le sel de potassium du lintitript ainsi obtenu.

Selon un aspect préféré, le procédé décrit ci-dessus est conduit à partir d'un 2-[[4-(2-chlorophényl)-2-thiazolyl]carbamoyl]indole-1-acétate de (C₁-C₄)alkyle, de préférence de l'ester méthylique.

La réaction à partir de l'acide 2-[[4-(2-chlorophényl)-2-thiazolyl]carbamoyl]indole-1-acétique ou de ses esters alkyliques en C₁-C₄, est conduite en mélangeant le produit dans le solvant peu miscible à l'eau, ajoutant du carbonate de potassium dans l'eau et chauffant le mélange pendant 1 à 30 heures. Après refroidissement, le sel de potassium du lintitript précipité peut être isolé selon les techniques usuelles et éventuellement recristallisé pour obtenir un produit exceptionnellement pur.

Le procédé de la présente invention, mis en oeuvre sur le lintript libre ou sur un de ses esters alkyliques en C₁-C₄ isolés ou dans le mélange provenant de la réaction du 1-carboxyindolyl-1-acétate de (C₁-C₄)alkyle avec le 2-amino-4-(2-chlorophényl)thiazole, conduit en général à un sel hydraté dans lequel l'eau de cristallisation est bien déterminée. Le sel de potassium est en fait obtenu sous forme de dihydrate. Le sel anhydre peut être obtenu par déshydratation de l'hydraté.

Le lintitript potassique dihydrate constitue un aspect ultérieur de la présente invention.

Comme solvants peu miscibles à l'eau, on peut utiliser des solvants qui donnent avec de l'eau un système biphasique; par exemple des éthers, des alcools supérieurs, avantageusement, un butanol ou une (C₄-C₆)alcanone, telle que la methyléthylcétone ou la méthylisobutylcétone. Parmi les butanols, le 2-butanol et l'isobutanol sont des solvants particulièrement avantageux.

Lorsqu'on utilise le *n*-butanol en tant que solvant peu miscible à l'eau, il peut s'avérer qu'un solvate du lintitript potassique avec le butanol utilisé et éventuellement avec de l'eau précipite du milieu réactionnel. Ce solvate peut être isolé et transformé dans le lintitript potassique dihydrate à l'état pur. Lorsque, comme butanol, on utilise le *n*-butanol, on obtient le 2-[[4-(2-chlorophényl)-2-thiazolyl]carbamoyl]indole-1-acétate potassique monohydrate mono-*n*-butanolate qui precipite et qui est de préférence isolé pour permettre sa recristallisation dans l'eau et l'obtention du 2-[[4-(2-chlorophényl)-2-thiazolyl]carbamoyl]indole-1-acétate potassique dihydrate très pur.

Le 2-[[4-(2-chlorophényl)-2-thiazolyl]carbamoyl]indole-1-acétate potassique monohydrate mono-*n*-butanolate de formule (I) 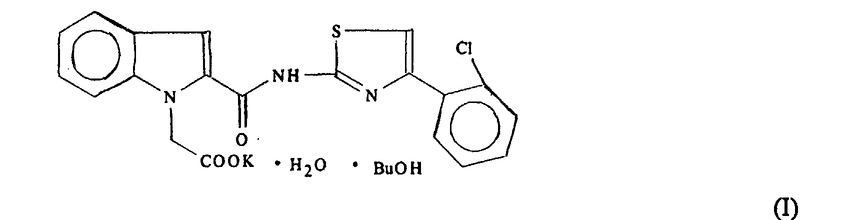 dans laquelle Bu est *n*-butyle, représente un objet ultérieur de la présente invention.

Par contre, lorsque l'on utilise le 2-butanol ou l'isobutanol, on n'obtient pas de solvate intermédiaire mais directement le lintitript potassique dihydrate, qui peut de toute façon être recristallisé dans l'eau si on souhaite obtenir un produit particulièrement pur.

Selon un autre de ses aspects, la présente invention concerne un procédé pour la préparation du sel de potassium du lintitript, caractérisé en ce qu'on traite un 2-carboxyindol-1-ylacétate de (C₁-C₄)alkyle de formule (II) 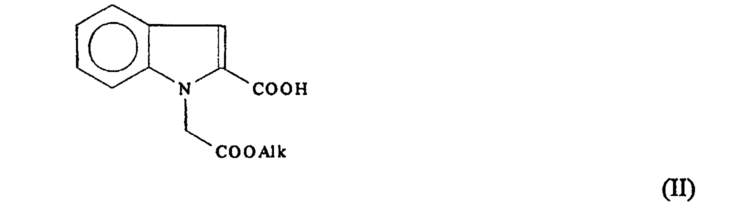 dans laquelle Alk représente un groupe (C₁-C₄)alkyle, avec le 2-amino-4-(2-chlorophényl)thiazole de formule (III), 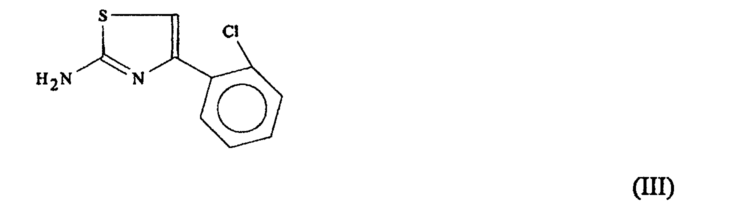 on saponifie *in situ* le 2-[[4-(2-chlorophényl)-2-thiazolyl]carbamoyl]indole-1-acétate de (C₁-C₄)alkyle ainsi obtenu avec du carbonate de potassium dans un système solvant biphasique constitué par un mélange d'eau et d'un solvant organique peu miscible à l'eau, éventuellement on recristallise dans l'eau le solvate intermediaire précipité et on isole le sel de potassium du lintitript ainsi obtenu.

La réaction entre le 2-carboxyindol-1-ylacétate de (C₁-C₄)alkyle et le 2-amino-4-(2-chlorophényl)thiazole est conduite en activant l'acide, par exemple à l'aide de chlorure de thionyle en présence de pyridine ou de diméthylformamide, et en effectuant le couplage avec l'amine en présence d'un agent de couplage tel que la 4-diméthylaminopyridine (DMAP) et d'une base forte, par exemple la triéthylamine, dans un solvant opportun. Le composé ainsi obtenu est soit isolé selon les techniques habituelles, soit soumis directement à la réaction de saponification, cette option étant préférable du point de vue pratique et économique. Comme solvant de couplage on peut bien utiliser le tétrahydrofurane, le chlorure de méthylène, le dichlorométhane.

La préparation des 2-carboxyindol-1-ylacétate de (C₁-C₄)alkyle de formule (II) est décrite dans EP-A-0 432 040.

La préparation du 2-amino-4-(2-chlorophényl)thiazole de formule (III) est également décrite dans EP-A-0 432 040, ainsi que dans EP-A-0-192 998.

Aussi lorsque la saponification est effectuée sur un 2-[[4-(2-chlorophényl)-2-thiazolyl]carbarnoyl]indole-1-acétate de (C₁-C₄)alkyle présent dans le mélange provenant de la réaction du 1-carboxyindolyl-l-acétate de (C₁-C₄)alkyle avec le 2-amino-4-(2-chlorophényl)thiazole, de façon avantageuse on utilise la méthylisobutylcétone ou un butanol comme solvant peu miscible à l'eau.

Le lintitript potassique dihydrate, qui est obtenu avec des rendements élevés sous une forme cristalline dense, bien filtrable, et aisément utilisable pour la préparation de formes pharmaceutiques aussi bien pour l'administration orale que pour l'administration parentérale, présente des maxima d'absorption à l'infrarouge caractéristiques, notamment à 3635 cm⁻¹, 1660 cm⁻¹, 1595 cm⁻¹, 1550 cm⁻¹, 1310 cm⁻¹, 1285 cm⁻¹ et 745 cm⁻¹.

Le lintitript potassique dihydrate ayant les maxima d'absorption caractéristiques à l'infrarouge ci-dessus constitue un objet préférentiel de la présente invention.

Le lintitript potassique dihydrate a été également caractérisé par diffractométrie des rayons X du monocristal avec un diffractomètre Rigaku AFC6S, graphite-monochromateur, source Cu-kα. Les données cristallographiques, notamment les distances interplanaires (a, b, c) les angles (α, β, γ) et le volume de chaque cellule unitaire sont les suivantes:

| | | |
|---|---|---|
| a = 10.453 (2) Å | b = 13.171 (2) Å | c = 8.253 (1) Å |
| α = 107.89 (1) | β = 100.96 (1) | γ = 89.74 (2) |
| vol = 2631.44 Å³ | | |
| Z=8 | | |

Les exemples qui suivent illustrent l'invention.

### EXEMPLE 1

### 2-[[4-(2-chlorophényl-2-thiazolyl]carbamoyl]indole-1-acétate potassique dihydrate

On chauffe au reflux pendant 3 heures 9,3 g (21,9 mmoles) de 2-[[4-(2-chlorophényl)-2-thizolyl]carbamoyl-indole-1-acétate de méthyle et 9,3 g (67,4 mmoles) de K₂CO₃ dans 60 ml d'isobutanol et 40 ml d'eau. Après refroidissement, on filtre le précipité et on le rince à l'isobutanol et à l'eau. On obtient le composé du titre. P.f. 263-266°C (déc.)

### EXEMPLE 2

### 2-[[4-(2-chlorophényl-2-thiazolyl]carbamoyl]indole-1-acétate potassique dihydrate

On chauffe au reflux pendant 15 heures un mélange de 170 g de 2-[[4-(2-chlorophényl)-2-thizolyl]carbamoyl-indole-1-acétate de méthyle dans 1 l de méthyléthylcétone avec 500 ml d'eau et 2,8 équivalent de K₂CO₃. Après refroidissement on filtre le précipité et on le rince à la méthyléthylcétone. On obtient le composé du titre. P.f. 263-266°C (déc.)

### EXEMPLE 3

### 2-[[4-(2-chlorophényl-2-thiazolyl]carbamoyl]indole-1-acétate potassique dihydrate

On chauffe au reflux pendant 3 heures 13,9 g (32,7 mmoles) de 2-[[4-(2-chlorophényl)-2-thizolyl]carbamoyl-indole-1-acétate de méthyle avec 15 g de K₂CO₃ dans 50 ml de méthylisobutylcétone et 50 ml d'eau. Après refroidissement, on filtre le précipité et on le rince à l'isobutanol et à l'eau. On obtient le composé du titre.

### EXEMPLE 4

### 2-[[4-(2-chlorophényl-2-thiazolyl]carbamoyl]indole-1-acétate potassique monohydrate mono-n-butanolate

On dissout 204,5 (0,50 mole) de 2-[[4-(2-chlorophényl)-2-thiazolyl]carbamoyl]indole-1-acétate de méthvle dans 500 ml de 1-butanol et on y ajoute une solution de 208 g (1,5 mole) de carbonate de potassium dans 700 ml d'eau. La suspension est étêtée jusqu'à 93°C en température de masse, puis chauffée au reflux pendant deux heures. On filtre le précipité et on le lave avec 400 ml de butanol, puis avec 900 ml d'eau. On obtient le composé du titre. P.f. 250-260 °C (déc.)

### EXEMPLE 5

### 2-[[4-(2-chlorophényl-2-thiazolyl]carbamoyl]indole-1-acétate potassique dihydrate

On chauffe 100 g du composé de l'exemple 1 dans 500 ml d'eau. On laisse refroidir la solution et on filtre le précipité formé. On obtient 85,58 g du composé du titre. P.f. 263-266°C (déc.)

### EXEMPLE 6

### 2-[[4-(2-chlorophényl-2-thiazolyl]carbamoyl]indole-1-acétate potassique dihydrate

On recristallise 9 g de produit de l'exemple 2 dans 60 ml de 1-butanol à 18% d'eau. On recristallise ensuite le précipité formé, sans séchage intermédiaire, dans 90 ml d'eau, sans étêtage. On obtient le composé du titre sous forme particulièrement pure, à 99,95%.

### EXEMPLE 7

### 2-[[4-(2-chlorophényl-2-thiazolyl]carbamoyl]indole-1-acétate potassique dihydrate

On chauffe au reflux pendant 3 heures 9,3 g (21,9 mmoles) de 2-[[4-(2-chlorophényl)-2-thizolyl]carbamoyl-indole-1-acétate de méthyle et 9,3 g (67,4 mmoles) de K₂CO₃ dans 60 ml de 2-butanol et 40 ml d'eau. Après refroidissement, on filtre le précipité et on le rince au 2-butanol et à l'eau. On obtient le composé du titre. P.f. 263-266°C (déc.).

## Revendications

1. Procédé pour la préparation du sel potassique dihydrate de l'acide 2-[[4-(2-chlorophényl)-2-thiazolyl]carbamoyl]indole-1-acétique, **caractérisé en ce que** l'on traite l'acide 2-[[4-(2-chlorophényl)-2-thiazolyl]carbamoyl]indole-1-acétique ou ses esters alkyliques en C₁-C₄ avec du carbonate de potassium dans un système solvant biphasique constitué par un mélange d'eau et d'un solvant organique peu miscible à l'eau, éventuellement on recristallise dans l'eau le solvate intermédiaire précipité et on isole le sel potassique dihydrate ainsi obtenu.

2. Procédé selon la revendication 1, **caractérisé en ce que**, la réaction est conduite à partir d'un 2-[[4-(2-chlorophényl)-2-thiazolyl]carbamoyl]indole-1-acétate de (C₁-C₄)alkyle.

3. Procédé selon la revendication 2, **caractérisé en ce que**, comme 2-[[4-(2-chlorophényl)-2-thiazolyl]carbamoyl]indole-1-acétate de (C₁-C₄)alkyle on utilise le 2-[[4-(2-chlorophényl)-2-thiazolyl]carbamoyl]indole-1-acétate de méthyle.

4. Procédé selon l'une quelconque des revendications 1 à 3, **caractérisé en ce que** le solvant peu miscible à l'eau est choisi parmi les éthers, les butanols et les (C₄-C₆)alcanones.

5. Procédé selon la revendication 4, **caractérisé en ce que**, comme solvant peu miscible à l'eau, on utilise la méthylisobutylcétone ou la méthyléthylcétone.

6. Procédé selon la revendication 4, **caractérisé en ce que** comme solvant peu miscible à l'eau on utilise un butanol.

7. Procédé pour la préparation du sel potassique dihydrate de l'acide 2-[[4-(2-chlorophényl)-2-thiazolyl]carbamoyl]indole-1-acétique, **caractérisé en ce qu'**on traite un 2-carboxyindol-1-ylacétate de (C₁-C₄)alkyle de formule (II) 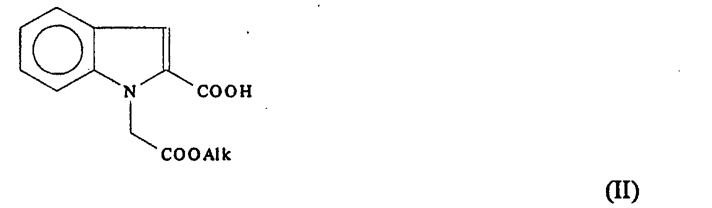 dans laquelle Alk représente un groupe (C₁-C₄)alkyle, avec le 2-amino-4-(2-chlorophényl)thiazole de formule (III), 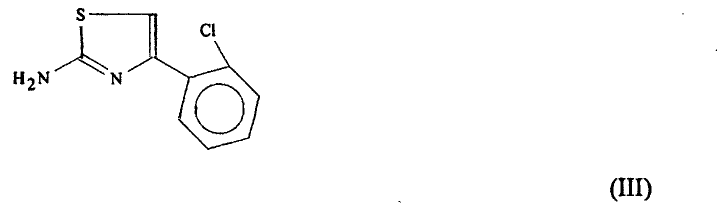 on saponifie *in situ* le 2-[[4-(2-chlorophényl)-2-thiazolyl]carbamoyl]indole-1-acétate de (C₁-C₄)alkyle avec du carbonate de potassium dans un système solvant biphasique constitué par un mélange d'eau et d'un solvant organique peu miscible à l'eau, éventuellement on recristallise dans l'eau le solvate intermédiaire précipité et on isole le sel de potassium dihydrate ainsi obtenu.

8. Procédé selon la revendication 7, **caractérisé en ce que**, comme 2-carboxyindolyl-1-acétate de (C₁-C₄)alkyle de départ, on utilise le 2-carboxyindolyl-1-acétate de méthyle.

9. Procédé selon la revendication 7 ou 8, **caractérisé en ce que**, comme solvant peu miscible à l'eau, on utilise la méthylisobutylcétone, la méthyléthylcétone ou un butanol.

10. Le 2-[[4-(2-chlorophényl)-2-thiazolyl]carbamoyl]indole-1-acétate potassique dihydrate.

11. Le 2-[[4-(2-chlorophényl)-2-thiazolyl]carbamoyl]indole-1-acétate potassique monohydrate mono-*n*-butanolate.

## Patentansprüche

1. Verfahren zur Herstellung des Kaliumsalz-Dihydrats der 2-[[4-(2-Chlorphenyl)-2-thiazolyl]-carbamoyl]-indol-1-essigsäure, **dadurch gekennzeichnet, daß** man die 2-[[4-(2-Chlorphenyl)-2-thiazolyl]-carbamoyl]-indol-1-essigsäure oder einen ihrer C₁-C₄-Alkylester mit Kaliumcarbonat in einem zweiphasigen Lösungsmittelsystem, welches aus einer Mischung aus Wasser und einem mit Wasser wenig mischbaren organischen Lösungsmittel gebildet ist, behandelt, das als Zwischenprodukt ausgefallene Solvat gegebenenfalls in Wasser umkristallisiert und das in dieser Weise erhaltene Kaliumsalz-Dihydrat isoliert.

2. Verfahren nach Anspruch 1, **dadurch gekennzeichnet, daß** die Reaktion ausgehend von einem 2-[[4-(2-Chlorphenyl)-2-thiazolyll-carbamoyll-indol-1-essigsäure-(C₁-C₄)-alkylester durchgeführt wird.

3. Verfahren nach Anspruch 2, **dadurch gekennzeichnet, daß** man als 2-[[4-(2-Chlorphenyl)-2-thiazolyl]-carbamoyl]-indol-1-essigsäure-(C₁-C₄)-alkylester 2-[[4-(2-Chlorphenyl)-2-thiazolyl]-carbamoyl]-indol-1-essigsäuremethylester verwendet.

4. Verfahren nach einem der Ansprüche 1 bis 3, **dadurch gekennzeichnet, daß** das mit Wasser wenig mischbare Lösungsmittel aus Estern, Butanolen und (C₄-C₆)-Alkanonen ausgewählt wird.

5. Verfahren nach Anspruch 4, **dadurch gekennzeichnet, daß** man als mit Wasser wenig mischbares Lösungsmittel Methylisobutylketon oder Methylethylketon verwendet.

6. Verfahren nach Anspruch 4, **dadurch gekennzeichnet, daß** man als mit Wasser wenig mischbares Lösungsmittel Butanol verwendet.

7. Verfahren zur Herstellung des Kaliumsalz-Dihydrats der 2-[[4-(2-Chlorphenyl)-2-thiazolyl]-carbamoyl]-indol-1-essigsäure, **dadurch gekennzeichnet, daß** man einen 2-Carboxyindol-2-yl-essigsäure-(C₁-C₄)-alkylester der Formel (II) 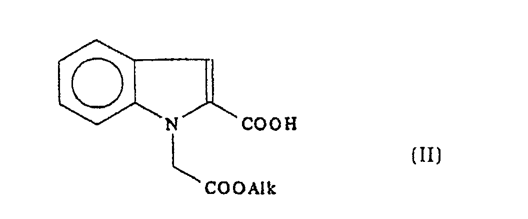 in der Alk eine (C₁-C₄)-Alkylgruppe darstellt, mit 2-Amino-4-(2-chlorphenyl)-thiazol der Formel (III) 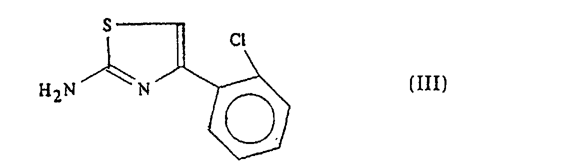 behandelt, den 2-[[4-(2-Chlorphenyl)-2-thiazolyl]-carbamoyl]-indol-1-essigsäure-(C₁-C₄)-alkylester *in situ* mit Kaliumcarbonat in einem zweiphasigen Lösungsmittelsystem, welches durch eine Mischung aus Wasser und einem mit Wasser wenig mischbaren organischen Lösungsmittel gebildet wird, verseift, gegebenenfalls das als Zwischenprodukt ausgefallene Solvat mit Wasser umkristallisiert und das in dieser Weise erhaltene Kaliumsalz-Dihydrat isoliert.

8. Verfahren nach Anspruch 7, **dadurch gekennzeichnet, daß** man als Ausgangs-2-Carboxyindolyl-1-essigsäure-(C₁-C₄)-alkylester 2-Carboxyindolyl-1-essigsäuremethylester verwendet.

9. Verfahren nach Anspruch 7 oder 8, **dadurch gekennzeichnet, daß** man als mit Wasser wenig mischbares organisches Lösungsmittel Methylisobutylketon, Methylethylketon oder ein Butanol verwendet.

10. 2-[[4-(2-Chlorphenyl)-2-thiazolyll-carbamoyl]-indol-1-essigsäure-Kaliumsalz-Dihydrat.

11. 2-[[4-(2-Chlorphenyl)-2-thiazolyl]-carbamoyl]-indol-1-essigsäure-Kaliumsalz-Monohydrat-Mono-*n*-butanolat.

## Claims

1. Process for the preparation of the potassium dihydrate salt of 2-[[4-(2-chlorophenyl)-2-thiazolyl]carbamoyl]indole-1-acetic acid, **characterized in that** 2-[[4-(2-chlorophenyl)-2-thiazolyl]carbamoyl]-indole-1-acetic acid or its C₁-C₄ alkyl esters is/are treated with potassium carbonate in a two-phase solvent system composed of a mixture of water and of an organic solvent which is not very miscible with water, the intermediate solvate precipitated is optionally recrystallized from water and the potassium dihydrate salt thus obtained is isolated.

2. Process according to Claim 1, **characterized in that** the reaction is carried out starting from a (C₁-C₄)alkyl 2-[[4-(2-chlorophenyl)-2-thiazolyl]-carbamoyl]indole-1-acetate.

3. Process according to Claim 2, **characterized in that** methyl 2-[[4-(2-chlorophenyl)-2-thiazolyl]-carbamoyl]indole-1-acetate is used as (C₁-C₄)alkyl 2-[[4-(2-chlorophenyl)-2-thiazolyl]carbamoyl]indole-1-acetate.

4. Process according to any one of Claims 1 to 3, **characterized in that** the solvent which is not very miscible with water is chosen from ethers, butanols and (C₄-C₆)alkanones.

5. Process according to Claim 4, **characterized in that** methyl isobutyl ketone or methyl ethyl ketone is used as solvent which is not very miscible with water.

6. Process according to Claim 4, **characterized in that** a butanol is used as solvent which is not very miscible with water.

7. Process for the preparation of the potassium dihydrate salt of 2-[[4-(2-chlorophenyl)-2-thiazolyl]carbamoyl]indole-1-acetic acid, **characterized in that** a (C₁-C₄)alkyl 2-carboxyindol-1-ylacetate of formula (II) 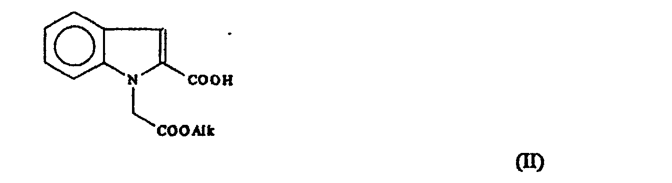 in which Alk represents a (C₁-C₄)alkyl group, is treated with 2-amino-4-(2-chlorophenyl)thiazole of formula (III) 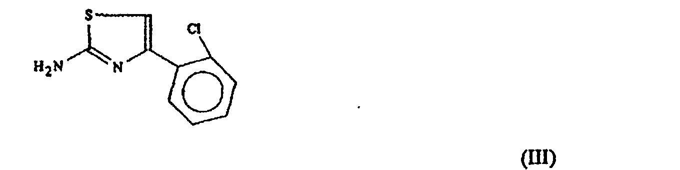 the (C₁-C₄)alkyl 2-[[4-(2-chlorophenyl)-2-thiazolyl]-carbamoyl]indole-1-acetate is saponified *in situ* with potassium carbonate in a two-phase solvent system composed of a mixture of water and of an organic solvent which is not very miscible with water, the intermediate solvate precipitated is optionally recrystallized from water and the potassium dihydrate salt thus obtained is isolated.

8. Process according to Claim 7, **characterized in that** methyl 2-carboxyindol-1-ylacetate is used as starting (C₁-C₄)alkyl 2-carboxyindol-1-ylacetate.

9. Process according to Claim 7 or 8, **characterized in that** methyl isobutyl ketone, methyl ethyl ketone or a butanol is used as solvent which is not very miscible with water.

10. Potassium 2-[[4-(2-chlorophenyl)-2-thiazolyl]-carbamoyl]indole-1-acetate dihydrate.

11. Potassium 2-[[4-(2-chlorophenyl)-2-thiazolyl]-carbamoyl]indole-1-acetate monohydrate mono-*n*-butanolate.
